# EUROPEAN PATENT APPLICATION

(11) **EP 3 572 041 A1**
(43) Date of publication of application: **27.11.2019**
(21) Application number: 18741500.5
(22) Date of filing: 29.01.2018
(51) Int. Cl.: A61F 2/16, A61L 27/14

(54) **INTRAOCULAR LENS AND METHOD FOR PRODUCING SAME**

(30) Priority: 20.01.2017 JP 2017008924
(71) Applicant: Kowa Company, Ltd., Nagoya-shi, Aichi 460-8625 (JP)
(72) Inventor: ISHIKAWA, Haruo, Nagoya-shi Aichi 457-0841 (JP); SHIMIZU, Norio, Nagoya-shi Aichi 457-0841 (JP)
(74) Representative: Ribeiro, James Michael
(86) International application number: PCT/IB2018/000036
(87) International publication number: WO 2018/134674

(57) **Abstract**

An intraocular lens whose application range is wider than that of the conventional single intraocular lens from the viewpoint of the characteristics at the time of bending of the support portion is made. An intraocular lens includes an optical portion having a predetermined refractive power; and a support portion configured to maintain a position of the optical portion in an eye. The support portion includes a main body formed of a flexible material and a shaft portion formed of an elastic material. At least a portion of the shaft portion is covered by the main body. An end of the shaft portion is provided away from the optical portion.

## Description

### [Field]

The present invention relates to an intraocular lens used for the treatment of a cataract and a method of manufacturing the same.

### [Background]

When you have a cataract in the eye, the crystalline lens turns cloudy and the view is worse. Therefore, treatment is performed to remove the crystalline lens that has turned cloudy by surgery and compensate for the refractive power which the crystalline lens originally has with another optical element. At present, a method of inserting an artificial crystalline lens, a so-called intraocular lens, into the crystalline capsule is generally provided.

The intraocular lens is often made of a foldable acrylic flexible material, and the intraocular lens can be folded and inserted from an incision provided in the cornea or the sclera. In addition, the toric intraocular lens capable of reducing astigmatism is also put to practical use. The toric intraocular lens is an intraocular lens having a cylindrical refractive power equivalent to that of corneal astigmatism. The toric intraocular lens is required to be inserted with the cylindrical axis of the lens aligned with the astigmatism axis of the cornea, and have a structure that does not cause positional deviation even after surgery.

In addition, although there are various types of intraocular lens in accordance with the case of the patient's eye, they are basically constituted by an optical portion that compensates for refractive power and a support portion configured to fix the intraocular lens in the eye. An intraocular lens in which an optical portion and a support portion are integrated is referred to as a one-piece intraocular lens, and an intraocular lens in which a support portion of a material different from that of the optical portion is attached is referred to as a multi-piece intraocular lens.

There has been offered a technique of providing an intraocular lens that is as versatile as possible so as not to be susceptible to individual differences among patients (Patent Literature 1). In addition, there has been also offered a technique of reducing the burden on tissues in the patient's eye as much as possible (Patent Literatures 2 and 3).

### [Citation List]

### [Patent Literature]

Patent Literature 1: JP 2008-86508 A
Patent Literature 2: JP S61-87546 A
Patent Literature 3: JP H01-300948 A

### [Summary]

### [Technical Problem]

Conventionally, it is necessary to prepare the one-piece intraocular lens and the multi-piece intraocular lens so that they can be prescribed according to the condition of the patient's eye, taking into consideration the characteristics at the time of bending of the support portion. However, when various intraocular lenses are stored, disadvantages that the expiration date of the intraocular lens has passed, management of each intraocular lens is complicated, it is not easy to secure a storage place, and the like will occur. In addition, for the manufacturing company, disadvantages that a burden from the viewpoint of manufacturing control and cost increases will occur, for example, different equipment is required depending on the type of intraocular lens to be manufactured, and the number of operators and inventories increase.

The technique of the present disclosure has been made in view of the above-described circumstances, and an object thereof is to provide an intraocular lens whose application range is wider than the application range of the conventional single intraocular lens from the viewpoint of the characteristic at the time of bending of the support portion.

### [Solution to Problem]

The intraocular lens of the present disclosure includes an optical portion having a predetermined refractive power, and a support portion configured to maintain a position of the optical portion in the eye, wherein the support portion includes a main body formed of a flexible material and a shaft portion formed of an elastic material, wherein at least a portion of the shaft portion is covered by the main body, and wherein an end of the shaft portion is provided away from the optical portion. The support portion of the intraocular lens thus obtained is more elastic than the support portion of the conventional one-piece intraocular lens, and more flexible than the support portion of the conventional multi-piece intraocular lens, so that it is possible to make an intraocular lens whose application range to the eye of a patient is wider than the application range of any one of the conventional one-piece intraocular lens and multi-piece intraocular lens.

In addition, a moment of inertia of area of the main body may be a moment of inertia of area such that a restoration speed when the support portion returns to a natural state at a time of bending is a restoration speed between a restoration speed when a support portion formed only of the flexible material returns to a natural state at a time of bending and a restoration speed when a support portion formed only of the elastic material returns to a natural state at a time of bending. In addition, the shaft portion may extend to the distal end of the support portion and the joint portion. A region where the shaft portion extends includes a region of the support portion that is most bent at a time of bending. Furthermore, a thickness of a portion of the main body in an optical axis direction of the optical portion, the portion covering the shaft portion, may be a thickness such that a restoration speed when the support portion returns to a natural state at a time of bending is a restoration speed between a restoration speed when a support portion formed only of the flexible material returns to a natural state at a time of bending and a restoration speed when a support portion formed only of the elastic material returns to a natural state at a time of bending.

In addition, in a method of manufacturing an intraocular lens of the present disclosure, the intraocular lens includes an optical portion having a predetermined refractive power and a support portion configured to maintain a position of the optical portion in the eye. The method includes forming the support portion including a main body formed of a flexible material and a shaft portion formed of an elastic material, covering at least a portion of the shaft portion by the main body, and providing an end of the shaft portion away from the optical portion. Alternatively, a monomer of the flexible material may be polymerized to form a guide configured to position the elastic material, and after the elastic material is positioned by the guide, a monomer of the flexible material may be polymerized to form the main body. Alternatively, the elastic material may be placed on a cross-link portion of a mold having the cross-link portion configured to cross-link the elastic material. After the elastic material is placed on the cross-link portion, a monomer of the flexible material may be polymerized to form the main body. Furthermore, after the main body is formed, alignment may be performed such that the elastic material is included in the support portion and the polymerized monomer of the flexible material may be cut out.

### [Advantageous Effects of Invention]

According to the technology of the present disclosure, an intraocular lens whose application range is wider than that of the conventional single intraocular lens from the viewpoint of the characteristics at the time of bending of the support portion may be made.

### [Brief Description of Drawings]

Figs. 1(a) and 1(b) are schematic views showing an example of an intraocular lens according to an embodiment.
Fig. 2 is a schematic diagram showing a modification of the intraocular lens.
Fig. 3 is a graph showing an example of a stress at the time of compression in the support portion of the intraocular lens according to an embodiment.
Figs. 4(a) and 4(b) are schematic configuration diagrams showing a measuring device that measures a stress at the time of compression in the support portion of the intraocular lens according to an embodiment.
Figs. 5(a) to 5(c) are diagrams schematically showing an example of a method of manufacturing the intraocular lens according to an embodiment.
Figs. 6(a) to 6(c) are diagrams schematically showing an example of a method of manufacturing the intraocular lens according to an embodiment.
Figs. 7(a) and 7(b) are diagrams schematically showing an example of a method of manufacturing the intraocular lens according to a modification.
Figs. 8(a) and 8(b) are schematic views showing an example of the intraocular lens according to a modification.

### [Description of Embodiments]

Hereinafter, embodiments of the present invention will be described. In the following description, the technical scope of the present invention is not limited to the following embodiments.

There are various types of intraocular lenses according to the case of a patient's eye, and can be roughly classified into two types of intraocular lenses: a one-piece intraocular lens and a multi-piece intraocular lens. An intraocular lens in which an optical portion and a support portion are integrated is referred to as a one-piece intraocular lens, and an intraocular lens in which a support portion of a material different from that of the optical portion is attached is referred to as a multi-piece intraocular lens. The one-piece intraocular lens has the following advantages and disadvantages as compared with the multi-piece intraocular lens since it has the integrated optical portion and support portion.

### (Advantages of one-piece intraocular lens)

- It is possible to manufacture the lens with one kind of material.
- It is easy to perform the production by the so-called molding method.
- Since there is no need to attach the support portion in the later process, the manufacturing process can be simplified and the production cost can be reduced easily.
- It is easy to insert the lens into a small incision since it is easy to design an intraocular lens with a thin optical portion and a thin support portion.
- There is little concern that the support portion may damage ocular tissue due to the high flexibility of the support portion.
- Since the support portion is wide, the contact area between the intraocular lens and the crystalline capsule is large and the position of the intraocular lens in the capsule is unlikely to be displaced when the intraocular lens is inserted into the eye.

### (Disadvantages of one-piece intraocular lens)

- Since the elasticity of the support portion is low, and the force to press the ocular tissue due to the repulsive force when the support portion is bent is small, the force to fix the position of the intraocular lens in the eye is weak.
- Since the force to press the optical portion against the posterior capsule by the support portion is weak, the adhesion of the edge of the optical portion to the posterior capsule is low, and the effect of suppressing the secondary cataract is low.
- It is difficult to perform repositioning by dialing or the like after the intraocular lens is inserted into the eye because the stickiness of the support portion is high (Dialing means that after the intraocular lens is inserted into the eye, the intraocular lens around the optical axis of the optical portion is made to rotate in order to enhance the stability of the intraocular lens in the eye and efficiently remove the viscoelastic substance poured into the eye as a buffer material.).
- If there is a crack in the posterior capsule, this type of lens cannot be used because the intraocular lens cannot be stabilized in the eye by the support portion.
- Since there is a possibility that the support portion may break, it may be difficult to sew and fix the lens.
- If the crystalline lens is large, the support portion may not be sufficiently bent, and the intraocular lens may not be sufficiently fixed in the eye.
- Restoration from the folded state when the intraocular lens is inserted into the eye is delayed, which may stress the operator.
- Insufficient contact between the support portion and the crystalline capsule during surgery may cause the intraocular lens to rotate unexpectedly in the eye.

On the other hand, multi-piece intraocular lens has the following advantages and disadvantages as compared to one-piece intraocular lens. Here, the multi-piece intraocular lens refers to an intraocular lens in which two support portions are connected to one optical portion.

### (Advantages of multi-piece intraocular lens)

- Since the elasticity of the support portion is high, the stress at the time of compression of the support portion is large.
- Stickiness of the support portion is low.
- The support portion can be formed in a thin diameter.
- Since there is little concern that the intraocular lens sticks to the crystalline capsule in the eye, repositioning by dialing or the like is easy.
- Since a force with which the support portion presses the optical portion against the posterior capsule is large, the effect of suppressing the secondary cataract is high.
- Even if there is a crack in the posterior capsule, or the crystalline capsule or Zinn's zonule is fragile, the lens can be inserted into the eye without the need to sew the support portion and the ocular tissue.
- There is a low risk of breakage of the support portion and it is easy to fix the lens by sewing.
- Even if the crystalline lens is large, a large repulsive force can be obtained by slightly bending the support portion, so that the intraocular lens can be fixed in the eye.

### (Disadvantages of multi-piece intraocular lens)

- Since it is necessary to ensure that a portion where the support portion is attached to an optical portion at the time of manufacture has a sufficient thickness, it may be unsuitable as an intraocular lens inserted in a small incision.
- The attachment of the support portion to the optical portion may require an operation by a skilled operator.
- A device may be required to attach the support portion to the optical portion.
- When the intraocular lens is inserted into the eye, the folded support portion may be recovered unexpectedly, which may increase the burden on the patient's ocular tissue when an unskilled operator handles the intraocular lens.
- Since the adhesion to the crystalline capsule is low, in the case of a so-called toric intraocular lens for reducing astigmatism, the intraocular lens is easily rotated and displaced when it is aligned in the eye.

As described above, the one-piece intraocular lens and the multi-piece intraocular lens each have advantages and disadvantages. For example, when it is required to insert an intraocular lens into a small incision as much as possible, or when an operator who has little experience in surgery performs a surgery, the one-piece intraocular lens is used, and when the stability of the intraocular lens in the eye is required, or when the condition of the crystalline capsule is concerned from the viewpoint of the above-mentioned disadvantages, the multi-piece intraocular lens is used. In this way, it is necessary to select the intraocular lens depending on the condition of the patient's eye or the situation of the surgery.

Therefore, in the present embodiment, an intraocular lens having both the advantages of the one-piece intraocular lens and the advantages of the multi-piece intraocular lens, that is, having high elasticity and flexibility is provided.

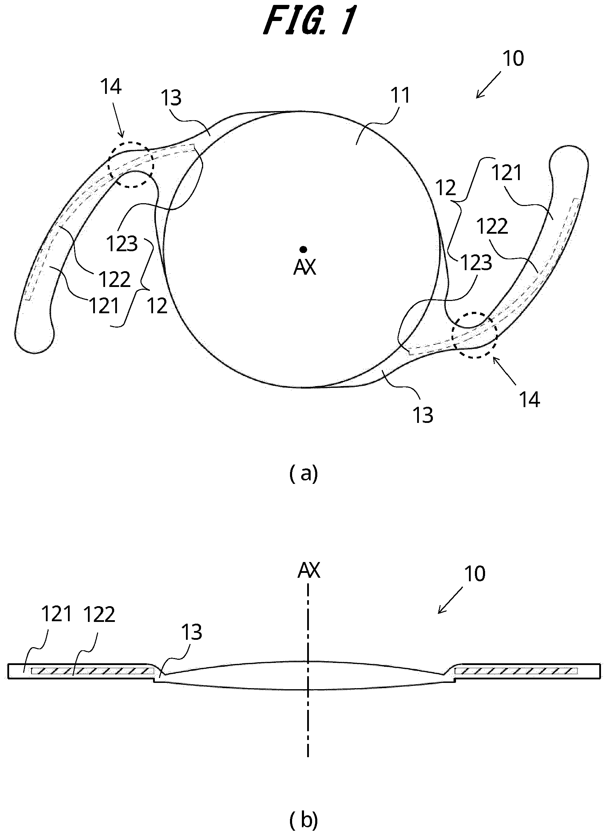

Fig. 1 is a schematic view showing an example of an intraocular lens 10 in the present embodiment. Fig. 1(a) is a view of the intraocular lens 1 when viewed in a direction parallel to the optical axis AX of an optical portion 11, and Fig. 1(b) is a view of the intraocular lens 1 when viewed in a direction perpendicular to the optical axis AX of the optical portion 11.

The intraocular lens 10 includes the optical portion 11 having a predetermined refractive power and two long flat plate-like support portions 12 configured to hold the optical portion 11 in the eye. Furthermore, the support portion 12 includes a main body 121 connected integrally with the optical portion 11. The support portion 12 also includes a shaft portion 122 covered by the main body 121. The optical portion 11 and the support portion 12 are connected to each other via a joint portion 13. The shaft portion 122 extends to the distal end of the support portion 12 and the joint portion 13. A region where the shaft portion 122 extends includes a region of the support portion 12 that is most bent when the support portion 12 is bent by receiving a compressive load such as coming into contact with the crystalline capsule.

As an example, in Fig. 1(a), a region 14 that is most bent when the support portion 12 is bent under a compressive load is indicated by a dotted circle. In the eye, it is probable that the external force applied to the support portion 12 is not local, but the well-rounded force is applied concentrically around the force point. From this point of view, it can be said that in the support portion 12, the region that is most bent before the external force is applied is the most flexible region. It is also probable that the region which is the most flexible in the support portion 12 be a region where the cross-sectional area of the flexible material (details are mentioned later) constituting the support portion 12 is the smallest.

A flexible material is used as a material of the optical portion 11 and the main body 121 of the support portion 12. Here, the flexible material refers to a material that is highly flexible and is not easily restored even when deformed. As an example, the flexible material includes a hydrophobic acrylic material, a hydrophilic acrylic material, or a silicone material. Further, an elastic material is used as a material of the shaft portion 122. Here, the elastic material refers to a material that is easily deformed but has a large repulsive force generated at the time of deformation and a high shape memory. As an example, the elastic material includes a polyvinylidene fluoride (PVDF) material, a rubber material, or a polyimide material.

In the intraocular lens 10 in the present embodiment, the end 123 of the shaft portion 122 is provided away from the optical portion 11. As long as the end 123 of the shaft portion 122 is away from the optical portion 11, the shaft portion 122 may be provided not to extend to the peripheral portion of the optical portion 11, that is, to the joint portion 13, which is a connection portion between the optical portion 11 and the support portion 12, or part of the shaft portion 122 may be provided to extend to the joint portion 13. In the conventional multi-piece intraocular lens, in order to connect the support portion to the optical portion, the thickness of the joint portion (corresponding to the joint portion 13 in the present embodiment) of the optical portion and the support portion is required to be secured, that is, it is necessary to secure a large cross-sectional area as viewed from the optical axis AX direction. On the other hand, according to the intraocular lens 10 in the present embodiment, since it is not necessary to connect the shaft portion 122 to the optical portion 11, the joint portion 13 has only to have a thickness sufficient to ensure the connection between the optical portion 11 and the main body 121. Therefore, the intraocular lens 10 in the present embodiment can have a joint portion thinner than the joint portion of the conventional multi-piece intraocular lens. Thereby, it can be said that the intraocular lens 10 can be inserted into a small incision as compared with the conventional multi-piece intraocular lens.

Next, the stress of the support portion 12 of the intraocular lens 10 at the time of compression will be described. In order to fix the intraocular lens in the eye, it is necessary for the intraocular lens to have sufficient force to push the ocular tissue. Since the intraocular lens cannot move autonomously, the intraocular lens is fixed in the eye using the force generated by receiving an external force, that is, the repulsive force of the support portion which is the elastic force of the support portion generated when the support portion bends or the stress at the time of compression of the support portion.

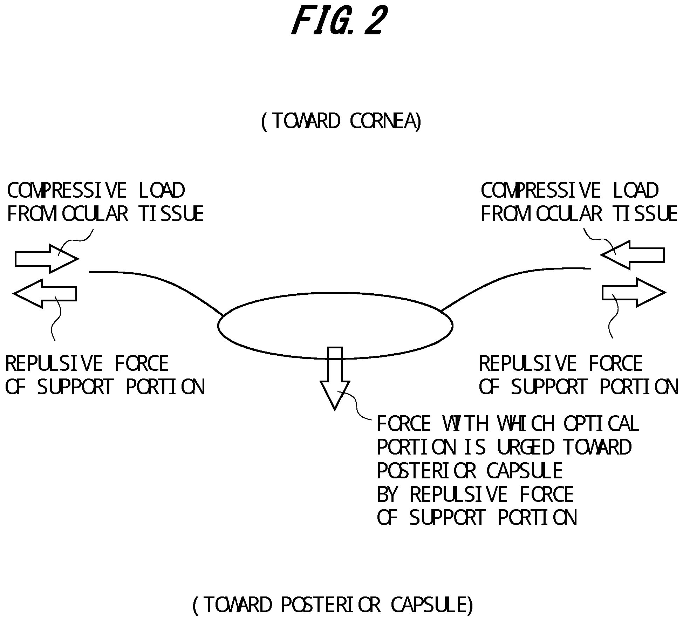

Fig. 2 schematically shows the force generated when the intraocular lens 10 is fixed in the eye. In the eye, the support portion 12 of the intraocular lens 10 comes into contact with the ocular tissue (such as a crystalline capsule) in a bent state. At this time, a compressive load is applied to the support portion 12 from the ocular tissue. The support portion 12 is subjected to a stress against a compressive load and an elastic force is generated by bending. Then, these stress and elastic force are generated as a repulsive force of the support portion 12 against the ocular tissue from the support portion 12. Moreover, as in a general intraocular lens, the support portion 12 of the intraocular lens 10 protrudes toward the cornea relative to the optical portion 11 in the eye. Therefore, when the support portion 12 of the intraocular lens 10 is compressed in the radial direction, that is, in the direction perpendicular to the optical axis of the optical portion 11, a force with which the optical portion 11 is urged toward the posterior capsule is generated by the above-described repulsive force of the support portion 12. Then, the optical portion 11 is urged toward the posterior capsule by the repulsive force of the support portion 12, whereby the intraocular lens 10 is fixed in the eye.

Thus, it can be said that whether the intraocular lens 10 is an intraocular lens that can be easily fixed in the eye depends on the magnitude of the elastic force and stress at the time of compression of the support portion 12. In general, the magnitude of the elastic force and stress at the time of compression of the support portion is larger in the multi-piece intraocular lens than in the one-piece intraocular lens. According to the intraocular lens 10 of the present embodiment, since the support portion 12 is configured to have the main body 121 and the shaft portion 122, the stress of the support portion 12 at the time of compression is larger than the stress of the support portion at the time of compression when the support portion 12 is constituted by only the main body 121, and smaller than the stress of the support portion at the time of compression when the support portion 12 is constituted by only the shaft portion 122 and the shaft portion 122 is directly connected to the optical portion. That is, the intraocular lens 10 of the present embodiment can have a stress larger than the stress at the time of compression by the support portion of the conventional one-piece intraocular lens, and smaller than the stress at the time of compression by the support portion of the conventional multi-piece intraocular lens.

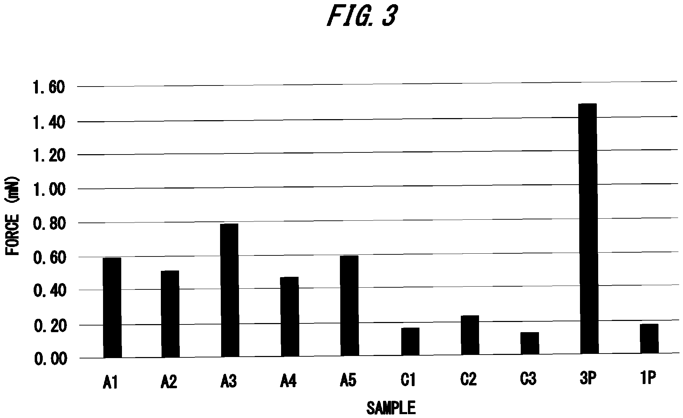

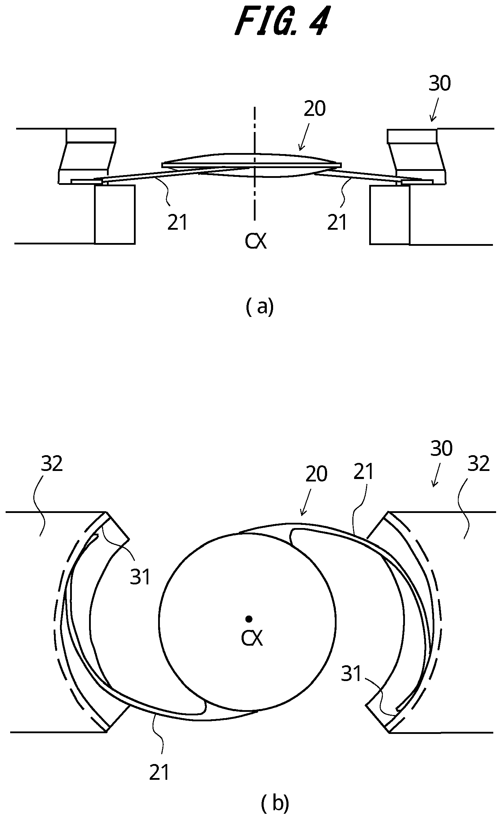

Fig. 3 shows an example of the graph in which the compressive loads at the time of compression are compared between the support portion 12 of a sample obtained by manufacturing the intraocular lens 10 in which the support portion 12 has the main body 121 and the shaft portion 122 as described above, a support portion of a sample of the conventional one-piece intraocular lens, and a support portion of a sample of the conventional multi-piece intraocular lens. Figs. 4 (a) and 4 (b) schematically show an example of a measurement method used to create the graph of Fig. 3. In this embodiment, the measurement is performed based on the compressive load test of ISO11979-3. In this measurement, the two support portions 21 of an intraocular lens 20 are brought into contact with a measuring device 30, and the intraocular lens 20 is positioned with respect to the measuring device 30. As shown in Fig. 4(b), the measuring device 30 has an arc-shaped surface 31 which simulates a general crystalline capsule in the eye, and part of the support portion 21 of the intraocular lens 20 comes into contact with the surface 31. Further, a main body 32 of the measuring device 30 can be moved in the direction perpendicular to the optical axis CX of the intraocular lens 20. By moving the main body 32, a compressive load according to the displacement of the main body 32 is applied to the support portion 21 from the surface 31. The main body 32 is connected to, for example, a load cell or the like, and it is possible to measure the stress from the support portion 21 caused by the deformation of the support portion 21 according to the displacement of the main body 32. The graph illustrated in Fig. 3 is obtained from the measured stress. In the following description, the repulsive force generated when the support portion receives an external force is referred to as a compressive load.

In the graph of Fig. 3, the numbers on the horizontal axis indicate the Sample Nos. Sample Nos. A1 to A5 indicate samples having the shaft portion 122 according to the present embodiment. Sample Nos. C1 to C3 indicate samples manufactured by the same method and not having the shaft portion 122. Sample No. "1P" indicates a sample of the conventional one-piece intraocular lens. Sample No. "3P" indicates a sample of the conventional multi-piece intraocular lens. Further, in the graph, the vertical axis represents the measured value (mN) of the compressive load. Since the sample in which the intraocular lens 10 is manufactured on a trial basis has a size such that the lens thickness of the optical portion 11 is 0.7 mm to 0.9 mm, the total length including the support portion and the optical portion is 13.0 mm, the thickness of the support portion is 0.35 mm, and the width of the support portion is 0.3 mm to 0.8 mm, which is equivalent to a size of the conventional one-piece intraocular lens, the intraocular lens of the present embodiment can be inserted into an incision with a size equivalent to a size when the conventional one-piece intraocular lens is inserted.

From the graph shown in Fig. 3, the support portion 12 of the samples of the intraocular lens 10 in the present embodiment whose Sample Nos. are A1 to A5 has a numerical value of the compressive load larger than that of the support portion of the conventional one-piece intraocular lens (Sample No "1P"). Further, the support portion 12 according to the samples having the Sample Nos. A1 to A5 has a numerical value of the compressive load smaller than that of the support portion of the conventional multi-piece intraocular lens. That is, the compressive load of the support portion 12 of the intraocular lens 10 of the present embodiment is between a compressive load of the support portion of the conventional one-piece intraocular lens and a compressive load of the support portion of the conventional multi-piece intraocular lens. From this, although the intraocular lens having the shaft portion in the present embodiment has an outer shape same as that of the conventional one-piece intraocular lens, the compressive load is large and it is probable that the fixation in the eye be improved. Furthermore, the disadvantage in that the lens is too elastic to handle as in the conventional multi-piece intraocular lens is improved.

Further, in the present embodiment, when viewed from the viewpoint of the moment of inertia of area of the flexible material forming the main body 121 of the support portion 12, it can be said that the main body 121 is formed to have the sufficient moment of inertia of area as compared with the support portion of the conventional one-piece intraocular lens. Here, the moment of inertia of area is a quantity that represents the inflexibility of deformation with respect to bending moment in a member such as a so-called beam member. As the cross section of the member changes, the value of the moment of inertia of area also changes. On the other hand, the samples C1 to C3, which are manufactured by the same manufacturing method, have a compressive load equivalent to that of the conventional one-piece intraocular lens, and if there is no shaft portion even when the manufacturing method of the present embodiment is used, an intraocular lens similar to the conventional one-piece intraocular lens can be made.

In addition, as in actual surgery, when the time for restoration from the folded state to the natural state with respect to the support portion of each sample of the intraocular lens in the present embodiment was visually checked, it was found that the time was shorter than the time for the support portion of the conventional one-piece intraocular lens, and longer than the time for the support portion of the conventional multi-piece intraocular lens.

Furthermore, the restoration speed of the support portion of each sample of the intraocular lens in the present embodiment was a speed at which the problem with the operation of the operator during the surgery would not occur. Moreover, the phenomenon in which the support portion of each sample of the intraocular lens in this embodiment was not restored to a natural state was not confirmed. It is probable that the stability of the intraocular lens in the eye in the present embodiment be higher than that of the conventional one-piece intraocular lens, considering that there is a possibility that restoration to the natural state may not occur due to the stickiness of the support portion in the conventional one-piece intraocular lens. Furthermore, since the volume of the entire intraocular lens in each sample of the intraocular lens in the present embodiment is equivalent to that of the conventional one-piece intraocular lens, it is probable that the insertion device for the conventional one-piece intraocular lens may be used for the intraocular lens in the present embodiment.

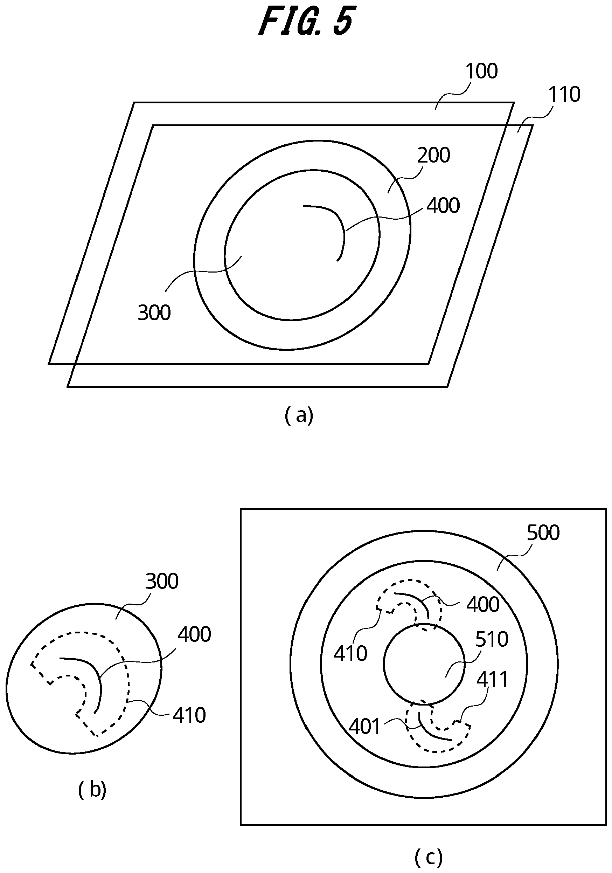

Next, an example of a method of manufacturing the intraocular lens 10 in the present embodiment will be described with reference to Figs. 5(a) to 5(c). In this manufacturing method, two transparent flat glass plates, a shim ring (thickness: 0.1 mm to 0.3 mm), a material described above as an elastic material, a resin mold used for manufacturing the conventional one-piece intraocular lens, and a monomer made of the material described above as a flexible material are used.

First, a shim ring is placed on a sheet of flat glass, and an elastic body is placed in the opening of the shim ring. The thickness of the shim ring is preferably larger than the thickness of the elastic material and not more than two thirds of the thickness of the support portion when the manufacture of the intraocular lens is completed. Also, the shim ring may be formed of a magnetic body.

Next, a monomer of a flexible material is poured into the opening of the shim ring, and another flat glass is placed on the shim ring so that the two flat glasses sandwich the shim ring. At this time, the placement is performed so as not to generate bubbles in the monomer. Also, the elastic material is prevented from coming into contact with the shim ring. When a shim ring is formed of a magnetic body, a shim ring can also be moved and aligned by using a magnet from on flat glass.

Next, the above-structured object is stored under a temperature environment where polymerization of the monomer of the flexible material is initiated, and the monomer is polymerized. Fig. 5(a) shows an example of the state of each member described above when polymerizing the monomer. As shown in Fig. 5(a), a shim ring 200, a monomer 300 of a flexible material, and a shaft portion 400 formed of an elastic material are sandwiched between two flat glasses 100 and 110. The shaft portion 400 formed of the elastic material is the shaft portion 122 of the support portion 12 when the manufacture of an intraocular lens is completed. At this time, by sandwiching and fixing the two flat glasses 100 and 110 by using magnets, polymerization of the monomer can be performed more reliably.

After polymerization of the monomers is completed, the sheet of polymerized monomer containing the shaft portion is cut into a suitable shape. As an example, as shown in Fig. 5(b), a sheet 410 including the shaft portion 400 is cut out in the shape shown by the dotted line from the monomer 300 which has been polymerized. Then, the sheet 410 which has been cut out is placed in the lower mold of the resin mold. There is a region provided for the support portion of the intraocular lens in the resin mold, and the sheet 410 is placed on the region. Here, with respect to the upper and lower sides of the sheet 410 to be placed, the sheet 410 is placed so that the exposed surface of the support portion is directed upward. Thus, the entire elastic material can be covered with the flexible material by pouring the monomer of the flexible material in the next step. The sheet 410 may be placed upside down, and, in this case, a portion of the elastic material wherein the portion is not covered with the flexible material is directed downward, so that even when the monomer of the flexible material is poured in the next step, the portion of the elastic material remains not covered with the flexible material. Whether a portion of the elastic material with respect to the sheet 410 wherein the portion is not covered with the flexible material is directed upward or downward may be determined as appropriate.

Fig. 5(c) shows an example of a state in which the sheets 410 and 411 including the elastic materials 400 and 401 are placed in a resin mold 500. The elastic material 401 and the sheet 411 are manufactured in the same manner as the elastic material 400 and the sheet 410. As shown in Fig. 5(c), the sheet 410 including the elastic material 400 is placed so as to sandwich a preparation region 510 of the optical portion of the intraocular lens. In the resin mold, in order to support alignment when placing the sheet 410, a protrusion or a bank may be provided in a portion which does not constitute the intraocular lens. Furthermore, a mark serving as a mark may be provided, for example, on a resin mold flange region so that the processing position can be distinguished when processing the outer shape of the intraocular lens.

Next, the monomer of the flexible material is poured into the inside of the resin mold, and covered with the upper mold of the resin mold and sealed. At this time, the placement is performed so as not to generate bubbles in the monomer. Further, the position of the sheet of the elastic material placed above is prevented from being pushed by the monomer and shifted. Then, the lens is stored under a temperature environment where the monomer of the flexible material is polymerized to complete the polymerization.

After polymerization of the monomer is completed, the intraocular lens is cut out from the resin mold according to the shape of the intraocular lens. At this time, alignment is performed so that the elastic material is included in the support portion. This alignment can be performed more easily if the above alignment mark is on the resin mold. The intraocular lens thus cut out is subjected to a process which is the same as the process of producing the conventional one-piece intraocular lens to complete the intraocular lens of the present embodiment.

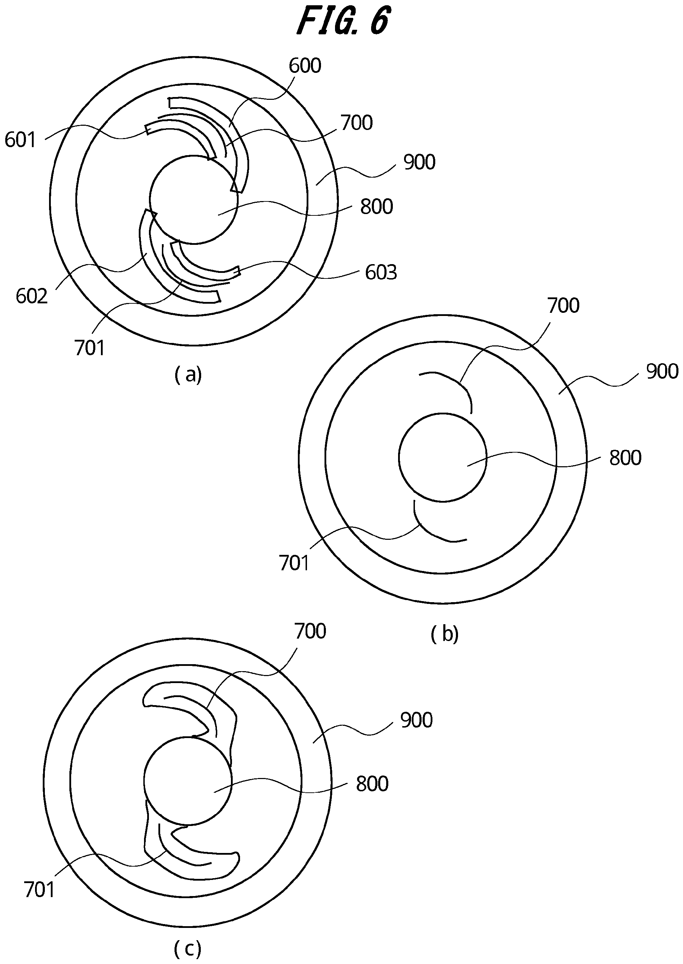

Next, referring to Figs. 6(a) to 6(c), an example of a method of manufacturing an intraocular lens according to the present embodiment will be described. In this manufacturing method, the resin mold (upper mold, lower mold) prepared to manufacture the intraocular lens of the present embodiment, the above-described material as an elastic material, and the monomer made of the material described above as a flexible material are used. Furthermore, the lower mold of the resin mold is provided with a mark for alignment of each material. Further, the upper mold of the resin mold is provided with a guide configured to position the elastic material forming the support portion.

First, in the lower mold of the resin mold, a guide configured to position the elastic material is formed by polymerizing the monomer of the flexible material. In the upper mold, a mold is formed to polymerize the monomer poured into the lower mold into the shape of the guide. The shape of the guide is such that it is easy to polymerize the monomer of the flexible material to be performed later, and it is possible to restrict the position of the elastic body. In addition, the guide is provided in the preparation region of the support portion in the lower mold. Further, the height of the guide from the mounting surface is set to be higher than the thickness of the elastic material and lower than the thickness of the main body of the intraocular lens to be manufactured. Furthermore, in the region where the elastic material is placed, a guide is formed so that the thickness of the polymerized flexible material is, for example, 0.1 mm or more. When polymerizing the monomer for a guide, a flexible material may exist in the preparation region of the optical portion in a lower mold. This is because the monomer polymerized for the guide also has the transparency of the finally obtained optical portion by mirror-finishing the surface of another upper mold that covers the monomers in the subsequent polymerization of the monomer of the flexible material. Next, the upper mold where the mold of the guide is formed is superimposed on the lower mold of the resin mold, and when polymerization of the monomer is completed and the guide is formed, the upper mold is removed from the lower mold, and the elastic body is placed on the lower mold using the guide.

Fig. 6(a) shows guides 600 to 603 formed of the flexible material on a lower mold 900 of the above resin mold, elastic materials 700 and 701 placed on the lower mold 900, and a flexible material 800 forming the optical portion. As shown in Fig. 6(a), the elastic materials 700 and 701 are placed so as to be sandwiched between the guides 600 to 603 when viewed from over the lower mold, and the elastic materials 700 and 701 are positioned by the guides 600 to 603. Since the elastic materials 700 and 701 are the shaft portion 122 of the intraocular lens 10 and are placed at a position away from the flexible material 800 forming the optical portion 11, the configuration is fabricated such that the shaft portion 122 in the intraocular lens 10 after manufacture is provided away from the optical portion 11.

Then, the monomer of the flexible material is poured, and the lower mold is covered with another upper mold to polymerize the monomer. Fig. 6(b) shows an example of a state in which the polymerization of the monomer is completed in the lower mold 900. The guides 600 to 603 formed in Fig. 6(a) disappear at this stage because the monomer is poured around them. After polymerization of the monomer is completed, alignment for cutting out the intraocular lens is performed, and then the outer shape of the intraocular lens is cut out. Fig. 6(c) shows an example of the intraocular lens to be cut out from the lower mold 900. As shown in Fig. 6(c), from the lower mold 900, the monomer in a region which is formed by polymerization and includes the elastic materials 700 and 701 and the flexible material 800 forming the optical portion is cut out. Further, the region surrounding the elastic materials 700 and 701 is cut out with a margin for forming the support portion 12 and the joint portion 13. The intraocular lens thus cut out is subjected to a process which is the same as the process of producing the conventional one-piece intraocular lens to complete the intraocular lens of the present embodiment.

The support portion 12 of the intraocular lens 10 obtained by the above-described manufacturing method includes the shaft portion 122 formed of the elastic material and the main body 121 formed of the flexible material 800. The main body 121 is provided so as to cover the shaft portion 122, and the joint portion 13 between the support portion 12 and the optical portion 11 is configured such that the shaft portion 122 is provided away from the optical portion 11, so that when the support portion 12 is bent, the resulting repulsive force is a repulsive force with a magnitude between a magnitude of the repulsive force of the support portion of the conventional one-piece intraocular lens and a magnitude of the repulsive force of the support portion of the conventional multi-piece intraocular lens. As a result, even if it is difficult to use the conventional one-piece intraocular lens or the conventional multi-piece intraocular lens when considering the disadvantages exemplified above, it is possible to use, from the viewpoint of the elastic force of the support portion and the stress at the time of compression, the intraocular lens 10 of the present embodiment as an intraocular lens that is more suitable for the conditions of the patient's eye.

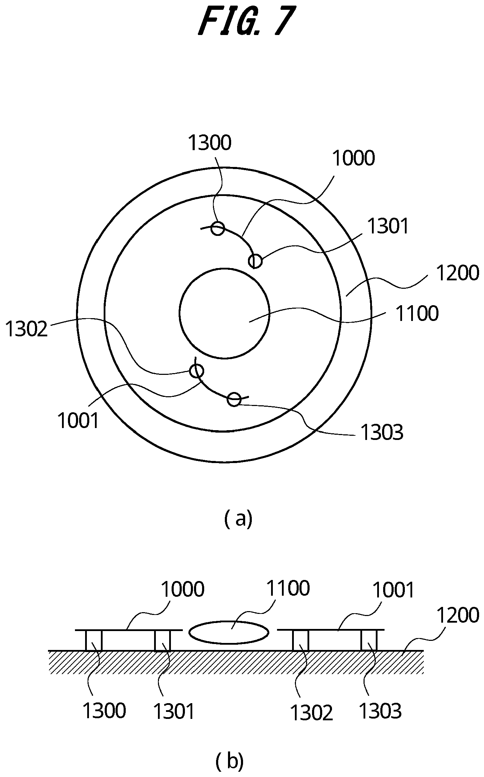

Although the above is a description regarding the present embodiment, the configuration of the above-mentioned intraocular lens is not limited to the above embodiment, and various modifications can be made without departing from the technical spirit of the present disclosure. In the following, the modification of the method of manufacturing in the above embodiment will be described with reference to Figs. 7(a) and 7(b).

As shown in schematic diagrams when viewed from over the lower mold and from the side of the lower mold in Figs. 7(a) and 7(b), in this modification, instead of the guides 600 to 603 described above, a lower mold 1200 is provided with cross-link portions 1300 to 1303 supporting the elastic material which is the shaft portion 122 of the support portions 12. The cross-link portions 1300 to 1303 can be formed, for example, by a method of polymerizing a monomer of a flexible material in the same manner as the guides 600 to 603. Then, an elastic material 1000 is placed on the cross-link portions 1300 and 1301, and an elastic material 1001 is placed on the cross-link portions 1302 and 1303. Thus, the intraocular lens can be manufactured by pouring and polymerizing the monomer of the flexible material in the same manner as described above in a state where the elastic materials 1000 and 1001 are placed on the cross-link portions 1300 to 1303.

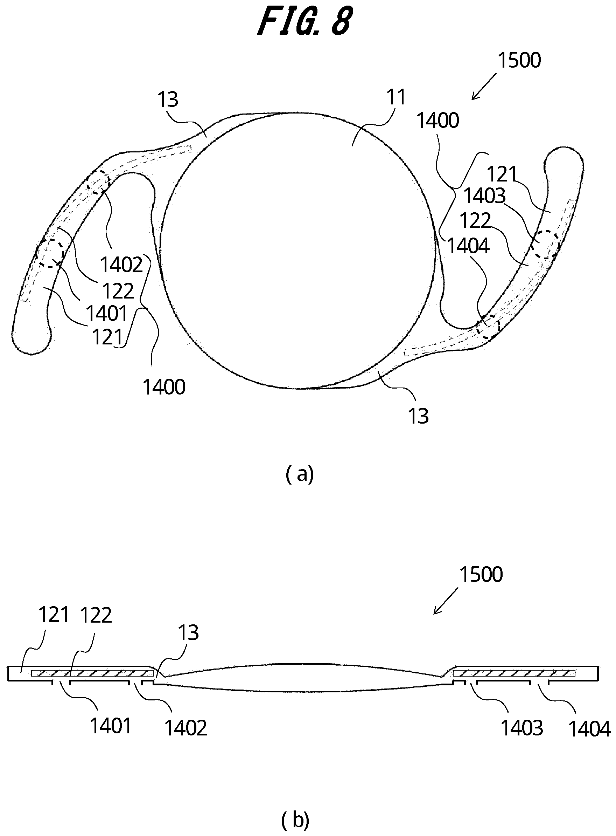

Figs. 8(a) and 8(b) show an example of an intraocular lens 1500 manufactured in this modification. The same components as those in the above-described embodiment are denoted by the same reference numerals, and detailed descriptions thereof will be omitted. In this modification, since the flexible material is not poured into a portion of the elastic materials 1000 and 1001 wherein the portion comes in contact with the cross-link portions 1300 to 1303, a support portion 1400 of the intraocular lens 1500 after manufacture has portions 1401 to 1404 of the shaft portion 122 wherein the portions are not covered by the main body 121. In this modification, the repulsive force such as stress at the time of compression of the support portion 1400 is changed by changing the number and size of the portions of the shaft portion 122 wherein the portions are not covered by the main body 121.

Further, the portions 1401 to 1404 of the shaft portion 122 wherein the portions are not covered by the main body 121 are provided in portions other than the portions which are bent most when the support portion 1400 is bent.

In the above description, although an example in which the optical surface is formed by a molding method is described, the optical surface may be formed by processing the optical surface after polymerization.

### [Reference Signs List]

- 10: intraocular lens
- 11: optical portion
- 12: support portion
- 121: main body
- 122: shaft portion
- 13: joint portion
- 500, 900, 1200: resin mold
- 1300 to 1303: cross-link portion
- 1401 to 1404: portion not covered by main body

## Claims

1. An intraocular lens comprising:
an optical portion having a predetermined refractive power; and a support portion configured to maintain a position of the optical portion in an eye,
wherein the support portion includes a main body formed of a flexible material and a shaft portion formed of an elastic material,
wherein at least a portion of the shaft portion is covered by the main body, and
wherein an end of the shaft portion is provided away from the optical portion.

2. The intraocular lens according to claim 1, wherein a moment of inertia of area of the main body is a moment of inertia of area such that a restoration speed when the support portion returns to a natural state at a time of bending is a restoration speed between a restoration speed when a support portion formed only of the flexible material returns to a natural state at a time of bending and a restoration speed when a support portion formed only of the elastic material returns to a natural state at a time of bending.

3. The intraocular lens according to claim 1 or 2, wherein the shaft portion extends to a distal end of the support portion and to the optical portion, a region where the shaft portion extends including a region of the support portion that is most bent at a time of bending.

4. The intraocular lens according to any one of claims 1 to 3, wherein a thickness of a portion of the main body in an optical axis direction of the optical portion, the portion covering the shaft portion, is a thickness such that a restoration speed when the support portion returns to a natural state at a time of bending is a restoration speed between a restoration speed when a support portion formed only of the flexible material returns to a natural state at a time of bending and a restoration speed when a support portion formed only of the elastic material returns to a natural state at a time of bending.

5. A method of manufacturing an intraocular lens including an optical portion having a predetermined refractive power and a support portion configured to maintain a position of the optical portion in an eye, the method comprising:
forming the support portion including a main body formed of a flexible material and a shaft portion formed of an elastic material;
covering at least a portion of the shaft portion by the main body; and
providing an end of the shaft portion away from the optical portion.

6. The method of manufacturing the intraocular lens according to claim 5, the method comprising:
forming a guide configured to position the elastic material by polymerizing a monomer of the flexible material; and
after the elastic material is positioned by the guide, polymerizing a monomer of the flexible material to form the main body.

7. The method of manufacturing the intraocular lens according to claim 5, the method comprising:
placing the elastic material on a cross-link portion of a mold having the cross-link portion configured to cross-link the elastic material; and
after the elastic material is placed on the cross-link portion, polymerizing a monomer of the flexible material to form the main body.

8. The method of manufacturing the intraocular lens according to claim 6 or 7, the method comprising: after forming the main body, performing alignment such that the elastic material is included in the support portion and cutting out the polymerized monomer of the flexible material.
